# EUROPEAN PATENT APPLICATION

(11) **EP 1 982 690 A2**
(43) Date of publication of application: **22.10.2008**
(21) Application number: 08103250.0
(22) Date of filing: 31.03.2008
(51) Int. Cl.: A61K 8/02, A61K 8/34, A61K 8/35, A61K 8/64, A61K 8/67, A61K 8/73, A61K 8/97, A61Q 19/08, A61K 9/70

(54) **Device for smoothing wrinkles on human skin**

(30) Priority: 18.04.2007 IT MI20070798
(71) Applicant: BIOFARMITALIA S.p.A., 20144 Milano (IT)
(72) Inventor: Pinna, Fausto, 20050 LESMO (MI) (IT); Pinna, Marco, 21051 ARCISATE (VA) (IT)
(74) Representative: Frignoli, Luigi

(57) **Abstract**

Device for smoothing wrinkles on human skin and for enabling skin sections between successive wrinkles to achieve prolonged contact with cosmetic and/or pharmaceutical substances carried by said device.

## Description

The present invention relates to a device for smoothing wrinkles on human skin and for enabling the skin in the cutaneous recesses or grooves between successive wrinkles to undergo prolonged cosmetic/therapeutic treatment.

For treating certain skin pathologies it is known to spread a suitable cream onto the skin or to resort to the use of a patch whose adhesive surface intended to be placed in contact with the skin carries one or more cosmetic or medical substances having the desired activity. Known patches for applying to the skin are poorly effective when applied in contact with a wrinkled skin, as they are unable to carry and hold for a sufficiently long time the active substances in contact with the skin in the cutaneous recesses or grooves between successive skin wrinkles.

Polypeptides are known to be substances with subcutaneous activity which act on the nerve endings to induce smoothing of wrinkles from within the skin, but these polypeptides find practical use only as creams or the like which remain in contact with the skin in the cutaneous grooves only for too short a time.

Substances are also known to exist having important anti-free radical properties, such as vitamin E and its derivatives, vitamin C, beta-carotene, plant extracts (such as polyphenols, bioflavonoids, various pigments), micronutrients (such as glutathione, selenium, coenzyme Q10 etc.) lipoic acid, glutathione, pycnogenol, sulphur-containing amino acids (such as cystine, cysteine, methionine), NAC, bioflavonoids, arginine, ornithine, OKG, glutamine, polyunsaturated fatty acids, lutein, lycopene, zeaxanthin, however the aforesaid considerations are also valid for these substances.

Many other substances, such as polyalcohols (glycerin), hyaluronic acid, ceramides, collagen and vegetable proteins have known and useful protective, nourishing and hydrating properties for the human skin.

For certain cosmetic and/or therapeutic applications (such as the curative treatment of wrinkles, scars and blemishes of the human skin) it would be highly desirable to maintain the aforementioned substances (and possibly others) in direct contact with the skin for a prolonged time, particularly with the depths of the cutaneous grooves between successive wrinkles. However, this is as yet an unresolved problem.

The main object of the present invention is therefore to provide an economical and easily used device which enables the active substances to be maintained in contact with the skin - particularly in the cutaneous grooves between successive wrinkles - for a relatively long time, even overnight or longer, thus enabling maximum effectiveness of the skin treatment.

Patent EP-B-1264552, the corresponding patent US-B-6761614 and patent WO 98/06360 in the name of the current applicant, the teachings of which are incorporated herein for reference, describe patches formed of a thin shaped sheet of a flexible material (meaning either a layer of synthetic material between 20 and 150 micron in thickness, or a porous or not porous fabric) to one surface of which a layer of skin-compatible adhesive, such as a water-based acrylic polymer is applied, and to the other surface of which an elongate bar is applied, made of semi-rigid synthetic material and having a thickness between 200 and 1000 microns, with only the two ends of the bar being fixed onto the adjacent surface of the shaped sheet.

Patent application EP-A-1655025 and the corresponding US2006/0093657A1 also in the name of the present applicants and whose teachings are incorporated herein for reference, describe, for application to the human skin, a patch having on one surface thereof a layer of skin-adhesive polymer, said adhesive consisting of a solvent-less polymer and polyvinyl alcohol, in said adhesive there being dispersed (in a quantity between 0.1% and 60% by weight) one or more substances having anti-inflammatory, cicatrising, emollient and other activities towards the skin.

The present invention represents an important advancement when compared to that described in the aforementioned patents and patent applications, as it relates to a device which enables wrinkles on human skin to be smoothed and the skin in the cutaneous grooves between successive wrinkles to be also subjected to prolonged treatment.

This device comprises a thin shaped sheet of synthetic flexible material and/or textile material, at least one elongate bar made of a semi-rigid synthetic material resting on one surface of the shaped sheet and welded thereto at the two ends of the bar, a layer of skin compatible polymer adhesive being applied on the other surface of the shaped sheet, characterised in that dispersed in said adhesive polymer there are at least one polypeptide and at least one active substance having anti-free radical properties chosen from the group consisting of vitamin E and its derivatives, vitamin C, beta-carotene, plant extracts (such as polyphenols, bioflavonoids, various pigments), micronutrients (such as glutathione, selenium, coenzyme Q10) lipoic acid, glutathione, pycnogenol, sulphur-containing amino acids (such as cystine, cysteine, methionine), NAC, bioflavonoids, arginine, ornithine, OKG, glutamine, polyunsaturated fatty acids, lutein, lycopene, zeaxanthin.

Preferably also at least one substance chosen from the group formed from polyalcohols, hyaluronic acid, ceramides, collagen and vegetable proteins is also dispersed in the adhesive polymer. Still more preferably, the adhesive polymer is chosen from the group formed from acrylic and/or vinyl polymers in an aqueous or solvent base, polyurethanes, resins of natural or synthetic origin, polyacrylates, natural polymers such as gums, polyvinyl alcohols, cellulose, carrageenans, alginates.

The active substance dispersed in the adhesive polymer is in a quantity between 0.1 % and 50% b.w. on the total weight of the adhesive polymer in which said substance is present.

The adhesive polymer can also usefully contain up to 5% by weight of a substance chosen from the group formed from wetting substances, solvents, preservatives, emulsifiers, stabilizers, solubilizers, surfactants and colorants.

The semi-rigid bars (which are fixed at their ends onto the free surface of the flexible shaped sheet) have a thickness of between 100 and 500 microns and are advantageously produced from a material chosen from the group formed from PVC, PET, PE, ABS and polystyrene.

Some non-limiting examples are described hereinafter to further clarify the understanding of the nature, shape and structure of the patch and of its preparation method, reference being made to the following drawings in which:
- Fig. 1 is a plan view of a punched thin piece of a polyurethane sheet;
- Fig. 2 is a plan view of a flexible elongate bar;
- Fig. 3 shows the bar of Fig. 2 rested on the upper surface of the sheet of Fig. 1, the ends of the bar being welded onto such sheet;
- Fig. 4 shows a longitudinal cross-section of the device, a layer of polymer adhesive being applied on the lower surface of the film and being protected by a thin sheet of siliconized paper;
- Figs. 5, 6 and 7 correspond to Figs. 1 to 3 but represent different shapes of the device and of its components.

### EXAMPLE 1

### Preparation of a wrinkle-smoothing device for the cosmetic treatment of wrinkles around the eyes and lips.

15.18 kg of non-crosslinked solvent-based acrylic adhesive (for example Duro-tak 387-2353 adhesive by National Starch & Chemical) are introduced into a container under cold conditions. In a separate container the cosmetic active components are prepared by combining 320 g of the skin exfoliating substances (alpha and beta-hydroxy acids), comprising 50 g of bilberry extract, 50 g of sugar cane extract, 50 g of maple extract, 100 g of sweet orange extract, 70 g of lemon extract, and 160 g of Indian fig extract, then mixing with a mixer (such as a Silverson Sp7) until a homogeneous mixture is obtained. 320 g of a substance with a lifting effect such a cork extract are added and agitation is continued until the mixture is completely homogeneous. The mixture of active substances is then poured into the flask containing the solvent-based acrylic adhesive and maintained under agitation using a propeller stirrer at about 120 rpm for 30 minutes, changing the height of the mixing rod every 10 minutes. It is left to stand to enable any air bubbles to escape. Using a patch spreading machine and with the aid of a compressed air pump, the mixture is fed into the rotating roller doctor blade with the doctor blade passage adjusted to about 260 microns. The mixture is filmed onto a siliconized paper of 70 g/m² which passes through four oven stations, the first set to 40° C, the second to 50° C, the third to 70° C and the fourth to 80° C, at a rate of 8 metres per minute. At the oven exit the film is completely free of solvents, which have evaporated within the oven stations; the amount of the adhesive mass is about 35 g/m². The siliconized paper on which the adhesive film was spread and dried is bonded to a polyurethane film of about 60 microns in thickness and rewound into a roll. The result is that the adhesive film grips the polyurethane protected by the siliconized paper.

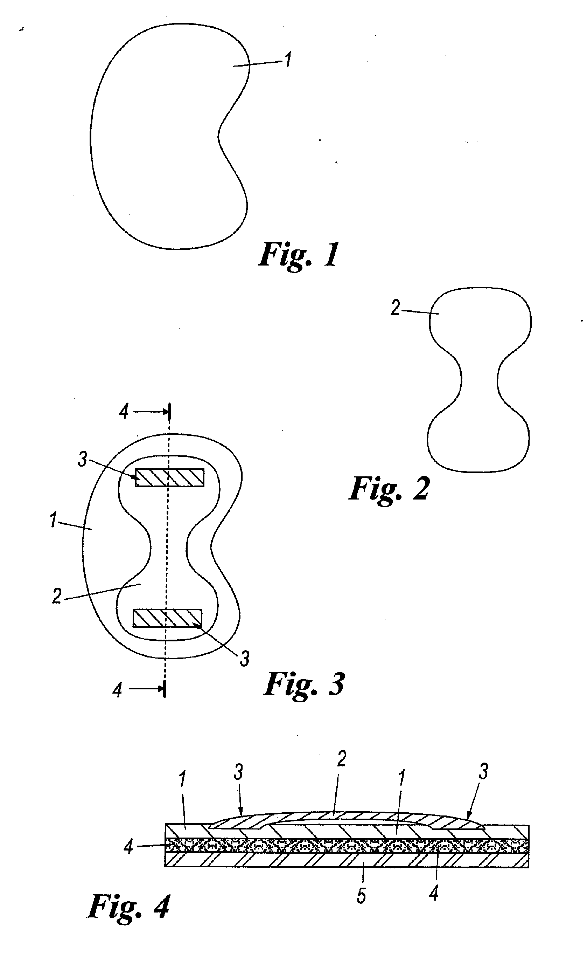

The roll obtained is then cut into rolls of suitable width to be punched to the shape shown in Figure 1 and indicated by the number 1, then (by means of a high frequency welding machine positioned at the punch exit) a flexible elongate bar 2 of 200 micron thick PVC (Figure 2) is applied to the surface of each punched element 1 without adhesive 4 and is welded at its free ends (in the regions indicated by the number 3) onto the adjacent free surface of the polyurethane support element.

In this manner a device is obtained such as that shown in cross-section in the accompanying Figure 4, formed from a shaped polyurethane sheet 1 (shown isolated in plan view in Figure 1) on the free surface of which a shaped bar 2 is rested (shown isolated in plan view in Figure 2), the ends 3 of which are welded onto the sheet 1: the bar 2 is made of PVC of 200 micron thickness. Figure 3 is a plan view of the device with the bar 2 resting and welded on the shaped sheet 1. On the other surface of the sheet 1 (i.e. on that surface facing downwards in Figure 4) the layer 4 of polymer adhesive is applied, protected by a thin sheet 5 of siliconized paper which is removed the moment the device is to be used. If the device is to be used to treat and remove the wrinkles from an eyelid, the eyelid is firstly held with two fingers to smooth the skin of the eyelid, then the device is applied to it so that it adheres thereto, while the bar 2 prevents the wrinkles from re-forming; in this manner the active substance present in the adhesive polymer can remaining contact with the surface of the skin at all points, for a prolonged time (for example overnight), to hence enable the active substance to totally develop its curative and cosmetic activity.

The wrinkle smoothing patch obtained in this manner is inserted into envelopes and boxes for sale.

### EXAMPLE 2

### Preparation of a wrinkle-smoothing device for the cosmetic treatment of forehead wrinkles

20 kg of 2500/3000 mPa.s medium viscosity cross-linked solvent-based acrylic adhesive of (for example Duro-tak 387-2054 adhesive by National Starch & Chemical) and 5 kg of 700-800 mPa.s low viscosity solvent-based acrylic adhesive are introduced under cold conditions into a container then mixed slowly for 20 minutes with a vertical agitator. While slowly agitating, 160 grams of fluid extract of red grape (Vitis vinifera), 120 grams of fluid extract of black bilberry (Vaccinium myrtillus), 80 grams of oily extract of soya (Glicine max) and 80 grams of fluid extract of sea buckthorn (Hippophae Rhamnoides) are introduced as a thin stream. The mixture is maintained under agitation using a propeller stirrer at about 120 rpm for 30 minutes, changing the height of the mixing every 10 minutes. It is left to stands to enable any air bubbles to escape. Using a patch spreading machine and with the aid of a compressed air pump, the mixture is fed into the rotating roller doctor blade with the doctor blade passage adjusted to about 260 microns. The mixture is filmed onto a siliconized paper of 70 g/m² which passes through four oven stations, the first set to 40° C, the second to 50° C, the third to 70° C and the fourth to 80° C, at a rate of 8 metres per minute. At the oven exit the film is completely free of solvents, which have evaporated within the oven stations; the amount of the adhesive mass is about 35 g/m². The siliconized paper on which the adhesive film was spread and dried is bonded to a polyurethane film of about 60 microns in thickness and rewound into a roll. The result is that the adhesive film grips the polyurethane protected by the siliconized paper.

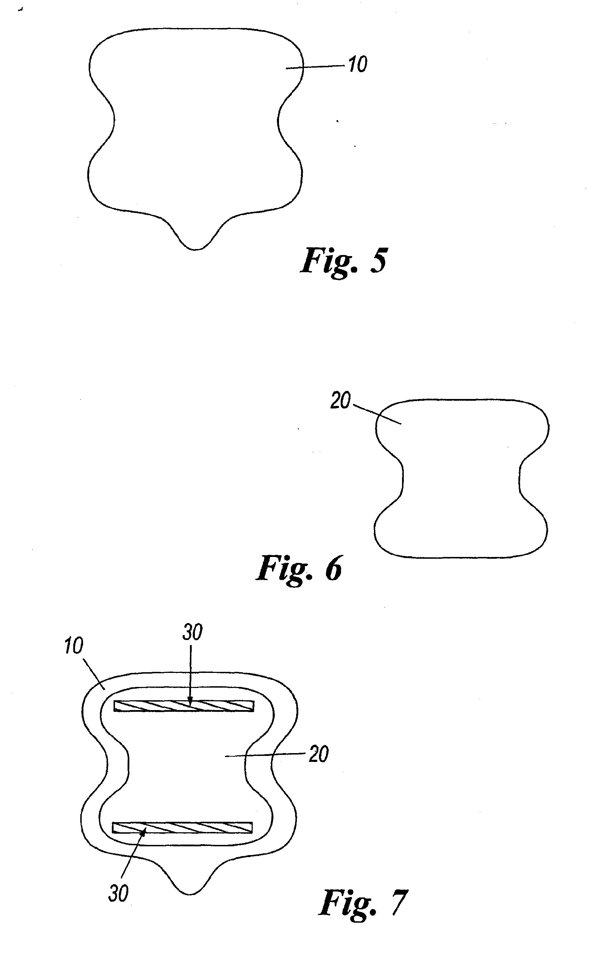

The roll obtained is then cut into rolls of suitable width to be punched to the shape shown in Figure 5 in which only the shaped polyurethane sheet 10 is shown in plan view, then by means of a high frequency welding machine positioned at the punch exit a tension bar 20 (shown in plan view in Figure 6) of 200 micron thick PVC is applied to each sheet 10 and is welded at its ends 30, to assume the appearance shown in plan in Figure 7. The cross-section through this device is similar to that shown in Figure 4, the use of the device to eliminate or treat the skin at the forehead wrinkles being also totally similar to that explained in Example 1.

The wrinkle smoothing device obtained in this manner is inserted into envelopes and boxes for sale.

### EXAMPLE 3

### Preparation of a wrinkle-smoothing device for the cosmetic treatment of lip wrinkles

50 kg of 2500/3000 mPa.s medium viscosity cross-linked solvent-based acrylic adhesive (for example Duro-tak 387-2054 adhesive by National Starch & Chemical) and 15 kg of 700-800 mPa.s low viscosity solvent-based acrylic adhesive are introduced under cold conditions into a container, then mixed slowly for 20 minutes with a vertical agitator. While slowly agitating, 50 grams of a substance active against nerve endings such as Esapeptide (for example Argireline, produced by Labotec), 80 grams of an anti-free radicals substance (for example 20 g of coenzyme Q10, 30 g of vitamin E and 30 g of glutathione), 120 grams of a hydrating substance (for example 30 g of glycerin, 30 g of hyaluronic acid and 60 g of maize protein extract) are introduced as a thin stream. The mixture is maintained under agitation using a propellor stirrer at about 120 rpm for 30 minutes, changing the height of the mixing rod every 10 minutes. It is left to stand to enable any air bubbles to escape. Using a patch spreading machine and with the aid of a compressed air pump, the mixture is fed into the rotating roller doctor blade with the doctor blade passage adjusted to about 260 microns. The mixture is filmed onto a siliconized paper of 70 g/m² which passes through four oven stations, the first set to 40° C, the second to 50° C, the third to 70° C and the fourth to 80° C, at a rate of 8 metres per minute. At the oven exit the film is completely free of solvents, which have evaporated within the oven stations; the amount of the adhesive mass is about 35 g/m². The siliconized paper on which the adhesive film was spread and dried is bonded to a polyurethane film of about 60 microns in thickness and rewound into a roll. The result is that the adhesive film grips the polyurethane protected by the siliconized paper. The roll obtained is then cut into rolls of suitable width to be punched to the shape shown in Figure 1, then by means of a high frequency welding machine positioned at the punch exit, the tension bar of 200 micron thick PVC (Figure 2) is applied to each patch, is welded at its ends, and shaped as in Figure 3. The wrinkle smoothing patch obtained in this manner is inserted into envelopes and boxes for sale.

## Claims

1. Device for smoothing wrinkles on human skin and for enabling cosmetic and/or pharmaceutical substances to remain in prolonged contact with skin in the cutaneous grooves between successive wrinkles, comprising a thin shaped sheet of flexible synthetic and/or textile material, on one surface of which at least one elongate bar made of a semi-rigid synthetic material is rested, its ends being welded to the adjacent surface of the shaped sheet, on the other surface of which a layer of skin compatible adhesive polymer is applied, **characterised in that** in said adhesive polymer an active substance comprising at least one polypeptide is dispersed, as is at least one active substance having anti-free radical properties chosen from the group consisting of vitamin E and its derivatives, vitamin C, beta-carotene, plant extracts, micronutrients, lipoic acid, glutathione, pycnogenol, sulphur-containing amino acids, NAC, bioflavonoids, arginine, ornithine, OKG, glutamine, polyunsaturated fatty acids, lutein, lycopene, zeaxanthin.

2. Device as claimed in claim 1, **characterised in that** at least one substance chosen from the group formed from polyalcohols, hyaluronic acid, ceramides, collagen and vegetable proteins is also dispersed in said adhesive polymer.

3. Device as claimed in claim 1 or 2, **characterised in that** said adhesive polymer is chosen from the group formed from acrylic and/or vinyl polymers in an aqueous or solvent base, polyurethanes, resins of natural or synthetic origin, polyacrylates, natural polymers such as gums, polyvinyl alcohols, cellulose, carrageenans, alginates.

4. Device as claimed in any one of claims 1, 2 or 3 **characterised in that** the quantity of active substance dispersed in the adhesive polymer is between 0.1% and 50% by weight on the total final weight of the adhesive polymer in which said active substance is present.
